# EUROPEAN PATENT SPECIFICATION

(11) **EP 3 621 697 B1**
(45) Date of publication and mention of the grant of the patent: **07.12.2022**
(21) Application number: 18722524.8
(22) Date of filing: 04.05.2018
(51) Int. Cl.: A61Q 5/06, A61K 8/891, A61K 8/898

(54) **DYE COMPOSITION BASED ON COPOLYMERS DERIVED FROM THE POLYMERIZATION OF AT LEAST ONE CROTONIC ACID MONOMER OR CROTONIC ACID DERIVATIVE AND OF AT LEAST ONE VINYL ESTER MONOMER AND ON SILICONE, AND PROCESS FOR DYEING KERATIN FIBERS USING THE SAME**
FARBSTOFFZUSAMMENSETZUNG AUF BASIS VON COPOLYMEREN ERHÄLTLICH DURCH POLYMERISATION VON MINDESTENS EINEM CROTONSÄUREMONOMER ODER EINEM CROTONSÄUREDERIVAT UND MINDESTENS VINYLESTERMONOMER UND AUF SILIKON, UND VERFAHREN ZUM FÄRBEN VON FÄRBEN VON KERATINFASERN UNTER VERWENDUNG DIESER ZUSAMMENSETZUNG
COMPOSITION TINCTORIALE À BASE DE COPOLYMÈRES ISSUS DE LA POLYMÉRISATION D'AU MOINS UN MONOMÈRE D'ACIDE CROTONIQUE OU D'UN DÉRIVÉ D'ACIDE CROTONIQUE ET D'AU MOINS UN MONOMÈRE D'ESTER VINYLIQUE ET DE SILICONE, ET PROCÉDÉ DE TEINTURE DES FIBRES KÉRATINIQUES LA METTANT EN OEUVRE

(30) Priority: 12.05.2017 FR 1754194
(43) Date of publication of application: 18.03.2020
(73) Proprietor: L'Oreal, 75008 Paris (FR)
(72) Inventor: SENECA, David, 93400 Saint-Ouen (FR); CHARRIER, Delphine, 93400 Saint-Ouen (FR); LASSALE, Melissa, 93400 Saint-Ouen (FR); BODELIN, Sophie, 94152 Chevilly La Rue (FR)
(74) Representative: L'Oreal
(86) International application number: PCT/EP2018/061575
(87) International publication number: WO 2018/206453

(56) References cited:
- EP-A2- 0 945 130
- FR-A1- 2 741 530
- FR-A1- 2 758 719
- US-A1- 2013 149 358
- US-A1- 2016 213 598
- DATABASE GNPD [Online] MINTEL; September 2016 (2016-09), "Hair spray", XP002773537, Database accession no. 4310851

## Description

The present invention relates to a process for dyeing keratin fibers, comprising the application of a composition containing at least one copolymer derived from the polymerization of at least one crotonic acid monomer or crotonic acid derivative and of at least one vinyl ester monomer, at least one amino silicone, the total amount of amino silicone(s) being at least 0.1% by weight relative to the total weight of the composition, and at least one pigment; and wherein the total amount of silicone(s) ranges from 0.1% to 15% by weight relative to the total weight of the composition.

In the field of dyeing keratin fibers, in particular human keratin fibers, it is already known practice to dye keratin fibers via various techniques using direct dyes or pigments for non-permanent dyeing, or dye precursors for permanent dyeing.

There are essentially three types of process for dyeing the hair:
a) "permanent" dyeing, the function of which is to afford a substantial modification to the natural color and which uses oxidation dyes which penetrate into the hair fiber and form the dye via an oxidative condensation process;
b) non-permanent, semi-permanent or direct dyeing, which does not use the oxidative condensation process and withstands four or five shampoo washes; it consists in dyeing keratin fibers with dye compositions containing direct dyes. These dyes are colored and coloring molecules that have affinity for keratin fibers.
c) temporary dyeing, which gives rise to a modification of the natural color of the hair that remains from one shampoo washing to the next, and which serves to enhance or correct a shade that has already been obtained. It may also be likened to a "makeup" process.

For this last type of dyeing, it is known practice to use colored polymers formed by grafting one or more dyes of azo, triphenylmethane, azine, indoamine or anthraquinone nature onto a polymer chain. These colored polymers are not entirely satisfactory, especially as regards the homogeneity of the coloring obtained and its resistance, not to mention the problems associated with their manufacture and especially with their reproducibility.

Another dyeing method consists in using pigments. Specifically, the use of pigment on the surface of keratin fibers generally makes it possible to obtain visible colorings on dark hair, since the surface pigment masks the natural color of the fiber. The use of pigment for dyeing keratin fibers is described, for example, in patent application FR 2 741 530; when they are applied to keratin fibers, these compositions have the drawback of transferring, i.e. of becoming at least partly deposited, leaving marks, on certain supports with which they may be placed in contact and in particular clothing or the skin. In addition, the product marketed under the name "Hair Spray" by Neez Profissional (GNPD database record ID:4310851) contains a VA/crotonates/vinyl neodecanoate copolymer, non-amino silicones and pigments. Also, the patent application US 2013/149358 discloses a process for dyeing the keratin fibers comprising the application of a composition containing at least one particular amino silicone and pigments.

This results in mediocre persistence of the applied film, making it necessary to regularly repeat the application of the composition. Moreover, the appearance of these unacceptable marks may put certain people off using this type of dyeing. Compositions for temporarily dyeing and/or making up the hair may also lead to a hair feel that is uncosmetic and/or not natural; the hair thus dyed may in particular lack softness and/or suppleness and/or individualization.

There is thus still a need to obtain compositions for the temporary dyeing of keratin materials, especially the hair, which have the advantage of forming a transfer-resistant deposit, which in particular does not become deposited, at least partly, onto supports with which said compositions are placed in contact, such as the skin (in particular the hands and the face) and/or clothing.

The invention is directed toward providing compositions which do not degrade keratin fibers, which do not impair their cosmetic properties such as softness and suppleness, keep the hair strands clearly individualized with no coarse feel, while at the same time having transfer-resistance properties.

This aim is achieved with the present invention, one subject of which is a process for dyeing keratin fibers, in particular human keratin fibers such as the hair, comprising the application to said fibers of a composition comprising (i) one or more copolymers derived from the polymerization of at least one crotonic acid monomer or crotonic acid derivative and of at least one vinyl ester monomer, (ii) one or more amino silicones, the total amount of amino silicone(s) ranging from 0.1% to 15% by weight relative to the total weight of the composition, and (iii) one or more pigments; and wherein the total amount of silicone(s) ranges from 0.1% to 15% by weight relative to the total weight of the composition.

A subject of the invention is also a cosmetic composition comprising (i) a crotonic acid/vinyl acetate/vinyl neodecanoate terpolymer, (ii) one or more amino silicones, the total amount of amino silicone ranging from 0.1% to 15% by weight relative to the total weight of the composition, and (iii) one or more pigments; and wherein the total amount of silicone(s) ranges from 0.1% to 15% by weight relative to the total weight of the composition.

The term "at least one" means "one or more".

The term "comprising a" means "comprising at least one", unless otherwise specified.

### Dye composition

The composition applied according to the invention is preferably a cosmetic composition for dyeing keratin fibers, in particular human keratin fibers such as hair.

It has been observed that by using the dye composition according to the invention, it is possible to improve the individualization of the hair strands, and also to reduce the transfer. The fibers also have a smoother feel, are softer and more supple, and disentangle more easily.

### Crotonic acid copolymers

The composition comprises at least one copolymer derived from the polymerization of at least one crotonic acid monomer or crotonic acid derivative and of at least one vinyl ester monomer, preferably at least two different vinyl ester monomers.

Preferably, the copolymer according to the invention is chosen from copolymers derived from the polymerization of at least one crotonic acid monomer and of at least one vinyl ester monomer, preferably at least two different vinyl ester monomers.

The term "crotonic acid derivative" preferably means a crotonic acid ester or a crotonic acid amide.

The term "crotonic acid derivative" preferably means a crotonic acid ester or amide, in particular:
- (i) the crotonic acid esters of formula CH₃CH=CHCOOR'1 with R'1 representing a linear, branched or cyclic, saturated or unsaturated, optionally aromatic (aryl, aralkyl or alkylaryl) carbon-based and especially hydrocarbon-based (alkyl) chain, containing 1 to 30 carbon atoms, optionally comprising one or more functions chosen from -OH, -OR' with R' C1-C6 alkyl (alkoxy), -CN, -X (halogen, especially Cl, F, Br or I); mention may be made, for example, of methyl crotonate and ethyl crotonate
- (ii) the crotonic acid amides of formula CH3CH=CHCONR'2R"2 with R'2 and R"2, which may be identical or different, representing hydrogen or a linear, branched or cyclic, saturated or unsaturated, optionally aromatic, carbon-based and especially hydrocarbon-based (alkyl) chain, containing 1 to 30 carbon atoms, optionally comprising one or more functions chosen from -OH, -OR' with R' C1-C6 alkyl (alkoxy), -CN, -X (halogen, especially Cl, F, Br or I).

The term "crotonic acid derivative" preferably means a crotonic acid ester or amide, in particular:
- (i) the crotonic acid esters of formula CH₃CH=CHCOOR'1 with R'1 representing a linear, branched or cyclic, saturated or unsaturated, optionally aromatic such as an aryl, aralkyl or alkylaryl, carbon-based and especially hydrocarbon-based chain such as an alkyl, containing 1 to 30 carbon atoms, optionally comprising one or more functions chosen from -OH, -OR' with R' C1-C6 alkyl such as an alkoxy, -CN, -X such as a halogen, especially Cl, F, Br or I; mention may be made, for example, of methyl crotonate and ethyl crotonate,
- (ii) the crotonic acid amides of formula CH3CH=CHCONR'2R"2 with R'2 and R"2, which may be identical or different, representing hydrogen or a linear, branched or cyclic, saturated or unsaturated, optionally aromatic, carbon-based and especially hydrocarbon-based chain such as an alkyl, containing 1 to 30 carbon atoms, optionally comprising one or more functions chosen from -OH, -OR' with R' C1-C6 alkyl such as an alkoxy, -CN, -X such as a halogen, especially Cl, F, Br or I.

The vinyl ester monomer(s) may be chosen from the compounds of formula CH2=CH-OCO-R'3 with R'3 representing a linear, branched or cyclic, saturated or unsaturated, optionally aromatic, carbon-based and especially hydrocarbon-based chain, containing 1 to 30 carbon atoms, optionally comprising one or more functions chosen from -OH, -OR' with R' C1-C6 alkyl (alkoxy), -CN, -X (halogen, especially Cl, F, Br or I).

Mention may be made especially of vinyl acetate, vinyl propionate, vinyl butyrate (or butanoate), vinyl ethylhexanoate, vinyl neononanoate, vinyl neododecanoate, vinyl neodecanoate, vinyl pivalate, vinyl cyclohexanoate, vinyl benzoate, vinyl 4-tert-butylbenzoate and vinyl trifluoroacetate.

Preferably, the copolymer according to the invention is chosen from copolymers derived from the polymerization of at least one crotonic acid monomer and of at least two different vinyl ester monomers, said vinyl ester monomers preferably being chosen from vinyl acetate, vinyl propionate, vinyl butyrate (or butanoate), vinyl ethylhexanoate, vinyl neononanoate, vinyl neododecanoate, vinyl neodecanoate, vinyl pivalate, vinyl cyclohexanoate, vinyl benzoate, vinyl 4-tert-butylbenzoate and vinyl trifluoroacetate, preferably from vinyl acetate, vinyl propionate and vinyl neodecanoate, better still from vinyl acetate and vinyl neodecanoate.

More particularly, the copolymer according to the invention is chosen from copolymers derived from the polymerization of crotonic acid, vinyl acetate and vinyl propionate, copolymers derived from the polymerization of crotonic acid, vinyl acetate and vinyl neodecanoate, and mixtures thereof.

According to a particular embodiment, the copolymer of the composition according to the invention is a crotonic acid/vinyl acetate/vinyl neodecanoate terpolymer.

The copolymers according to the invention may optionally comprise other monomers such as allylic or methallylic esters, or vinyl ethers. These polymers may optionally be grafted or crosslinked.

Such polymers are described, *inter alia,* in French patent Nos. 1 222 944, 1 580 545, 2 265 782, 2 265 781, 1 564 110 and 2 439 798. Commercial products which fall into this category are the products Resyn^{®} 28-2930 and 28-1310 sold by the company Akzo Nobel (INCI names VA / crotonates / vinyl decanoate copolymer and VA / crotonates copolymer, respectively). Mention may also be made of the products Luviset^{®} CA 66 sold by the company BASF, Aristoflex^{®} A60 sold by the company Clariant (INCI name VA / crotonates copolymer) and Mexomere^{®} PW or PAM sold by the company Chimex (INCI name VA / vinyl butyl benzoate / crotonates copolymer).

The total amount of copolymer(s) of crotonic acid or crotonic acid derivative according to the invention may range from 0.05% to 15% by weight relative to the weight of the composition, preferably from 0.1% to 10% by weight relative to the weight of the composition, preferably from 1% to 5% by weight relative to the weight of the composition.

### Silicones

As indicated above, the composition contains one or more silicones. Preferably, the composition contains at least two different silicones.

According to the invention, the composition contains at least one amino silicone.

The silicones may be solid or liquid at 25°C and atmospheric pressure (1.013×10⁵ Pa), and volatile or nonvolatile.

The silicones that may be used may be soluble or insoluble in the composition according to the invention; they may be in the form of oil, wax, resin or gum; silicone oils are preferred.

Silicones are especially described in detail in Walter Noll's Chemistry and Technology of Silicones (1968), Academic Press.

Preferably, the composition contains one or more silicones that are liquid at 25°C and atmospheric pressure (1.013×10⁵ Pa).

The volatile silicones may be chosen from those with a boiling point of between 60°C and 260°C (at atmospheric pressure) and more particularly from:
i) cyclic polydialkylsiloxanes comprising from 3 to 7 and preferably 4 to 5 silicon atoms, such as
   - octamethylcyclotetrasiloxane and decamethylcyclopentasiloxane.
      Mention may be made of the products sold under the name Volatile Silicone 7207 by Union Carbide or Silbione 70045 V 2 by Rhodia, Volatile Silicone 7158 by Union Carbide or Silbione 70045 V 5 by Rhodia;
   - cyclocopolymers of the dimethylsiloxane/methylalkylsiloxane type having the chemical structure: 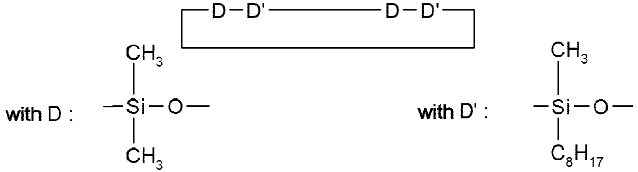 Preferably cyclomethylsiloxane.
      Mention may be made of Volatile Silicone FZ 3109 sold by the company Union Carbide;
   - mixtures of cyclic silicones with silicon-derived organic compounds, such as the mixture of octamethylcyclotetrasiloxane and of tetratrimethylsilylpentaerythritol (50/50) and the mixture of octamethylcyclotetrasiloxane and of 1,1'-oxy(2,2,2',2',3,3'-hexatrimethylsilyloxy)bisneopentane;
ii) linear polydialkylsiloxanes containing 2 to 9 silicon atoms, which generally have a viscosity of less than or equal to 5 ×10⁻⁶ m²/s at 25°C, such as decamethyltetrasiloxane.

Other silicones belonging to this category are described in the article published in Cosmetics and Toiletries, Vol. 91, Jan. 76, pages 27-32, Todd & Byers "Volatile Silicone Fluids for Cosmetics"; mention may be made of the product sold under the name SH 200 by the company Toray Silicone.

Among the nonvolatile silicones, mention may be made, alone or as a mixture, of polydialkylsiloxanes and especially polydimethylsiloxanes (PDMS), polydiarylsiloxanes, polyalkylarylsiloxanes, silicone gums and resins, and also organopolysiloxanes (or organomodified polysiloxanes, or alternatively organomodified silicones) which are polysiloxanes comprising in their structure one or more organofunctional groups, generally attached via a hydrocarbon-based group, and preferably chosen from aryl groups, amine groups, alkoxy groups and polyoxyethylene or polyoxypropylene groups. Preferably, the nonvolatile silicones are chosen from poly dimethyl/methylsiloxane which is optionally oxyethylenated and oxypropylenated.

The organomodified silicones may be polydiarylsiloxanes, in particular polydiphenylsiloxanes, and polyalkylarylsiloxanes, functionalized with the organofunctional groups mentioned previously. The polyalkylarylsiloxanes are particularly chosen from linear and/or branched polydimethyl/methylphenylsiloxanes and polydimethyl/diphenylsiloxanes.

Among the organomodified silicones, mention may be made of organopolysiloxanes comprising:
- polyethyleneoxy and/or polypropyleneoxy groups optionally comprising C₆-C₂₄ alkyl groups, such as dimethicone copolyols and especially those sold by the company Dow Corning under the name DC 1248 or the oils Silwet^{®} L 722, L 7500, L 77 and L 711 from the company Union Carbide; or (C₁₂)alkylmethicone copolyols and especially those sold by the company Dow Corning under the name Q2 5200;
- substituted or unsubstituted amine groups, in particular C₁-C₄ aminoalkyl groups; mention may be made of the products sold under the names GP4 Silicone Fluid and GP7100 by the company Genesee, or under the names Q2-8220 and DC929 or DC939 by the company Dow Corning;
- thiol groups, such as the products sold under the names GP 72 A and GP 71 from Genesee;
- alkoxylated groups, such as the product sold under the name Silicone Copolymer F-755 by SWS Silicones and Abil Wax^{®} 2428, 2434 and 2440 by the company Goldschmidt;
- hydroxylated groups, for instance polyorganosiloxanes bearing a hydroxyalkyl function;
- acyloxyalkyl groups, such as the polyorganosiloxanes described in patent US-A-4 957 732;
- anionic groups of the carboxylic acid type, as described, for example, in EP 186 507, or of the alkylcarboxylic type, such as the product X-22-3701E from the company Shin-Etsu; or else of the 2-hydroxyalkylsulfonate or 2-hydroxyalkylthiosulfate type, such as the products sold by the company Goldschmidt under the names Abil^{®} S201 and Abil^{®} S255;
- hydroxyacylamino groups, such as the polyorganosiloxanes described in patent application EP 342 834; mention may be made, for example, of the product Q2-8413 from the company Dow Corning.

The silicones may also be chosen from polydialkylsiloxanes, among which mention may be made mainly of polydimethylsiloxanes bearing trimethylsilyl end groups. Among these polydialkylsiloxanes, mention may be made of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 with a viscosity of 60 000 mm²/s;
- the Viscasil^{®} oils from General Electric and certain oils of the SF series (SF 96, SF 18) from General Electric.

Mention may also be made of polydimethylsiloxanes bearing dimethylsilanol end groups, known under the name dimethiconol (CTFA), such as the oils of the 48 series from the company Rhodia.

In this category of polydialkylsiloxanes, mention may also be made of the products sold under the names Abil Wax^{®} 9800 and 9801 by the company Goldschmidt, which are poly(C1-C20)dialkylsiloxanes.

Products that may be used more particularly in accordance with the invention are mixtures such as:
- mixtures formed from a polydimethylsiloxane with a hydroxy-terminated chain, or dimethiconol (CTFA), and from a cyclic polydimethylsiloxane, also known as cyclomethicone (CTFA), such as the product Q2 -1401 sold by the company Dow Corning.

The polyalkylarylsiloxanes are particularly chosen from linear and/or branched polydimethyl/methylphenylsiloxanes and polydimethyl/diphenylsiloxanes with a viscosity ranging from 1×10⁻⁵ to 5×10⁻² m²/s at 25°C.

Among these polyalkylarylsiloxanes, mention may be made of the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Preferably, the composition according to the invention contains at least one dimethicone.

The composition according to the invention comprises one or more amino silicones. The term "amino silicone" denotes any silicone comprising at least one primary, secondary or tertiary amine or a quaternary ammonium group.

The weight-average molecular masses of these amino silicones may be measured by gel permeation chromatography (GPC) at room temperature (25°C), as polystyrene equivalent. The columns used are µ styragel columns. The eluent is THF and the flow rate is 1 ml/min. 200 µl of a 0.5% by weight solution of silicone in THF are injected. Detection is performed by refractometry and UV-metry.

Preferably, the amino silicone(s) that may be used in the context of the invention are chosen from:
a) the polysiloxanes corresponding to formula (A): 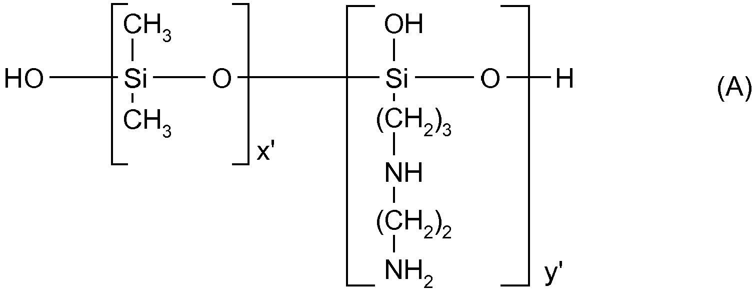 in which x' and y' are integers such that the weight-average molecular weight (Mw) is between 5000 and 500 000 approximately;
b) the amino silicones corresponding to formula (B):

   R'ₐG₃₋ₐ-Si(OSiG₂)ₙ-(OSiG_{b}R'_{2-b})ₘ-O-SiG₃₋ₐ-R'ₐ (B)

   in which:
   - G, which may be identical or different, denotes a hydrogen atom or a phenyl, OH, C₁-C₈ alkyl, for example methyl, or C₁-C₈ alkoxy, for example methoxy, group,
   - a, which may be identical or different, denotes 0 or an integer from 1 to 3, in particular 0,
   - b denotes 0 or 1, in particular 1,
   - m and n are numbers such that the sum (n + m) ranges from 1 to 2000 and in particular from 50 to 150, n possibly denoting a number from 0 to 1999 and in particular from 49 to 149, and m possibly denoting a number from 1 to 2000 and in particular from 1 to 10;
   - R', which may be identical or different, denotes a monovalent radical of formula -CqH2qL in which q is a number ranging from 2 to 8 and L is an optionally quaternized amine group chosen from the following groups:
      - N(R")₂; -N⁺(R")₃ A-; -NR"-Q-N(R")₂ and -NR"-Q-N⁺(R")₃ A-,
      in which R", which may be identical or different, denotes hydrogen, phenyl, benzyl, or a saturated monovalent hydrocarbon-based radical, for example a C1-C20 alkyl radical; Q denotes a linear or branched group of formula CᵣH₂ᵣ, r being an integer ranging from 2 to 6, preferably from 2 to 4; and A- represents a cosmetically acceptable anion, in particular a halide such as fluoride, chloride, bromide or iodide.

Preferably, the amino silicones are chosen from the amino silicones of formula (B). Preferably, the amino silicones of formula (B) are chosen from amino silicones corresponding to formulae (C), (D), (E), (F) and/or (G) below.

According to a first embodiment, the amino silicones corresponding to formula (B) are chosen from the silicones known as "trimethylsilyl amodimethicone" corresponding to formula (C): 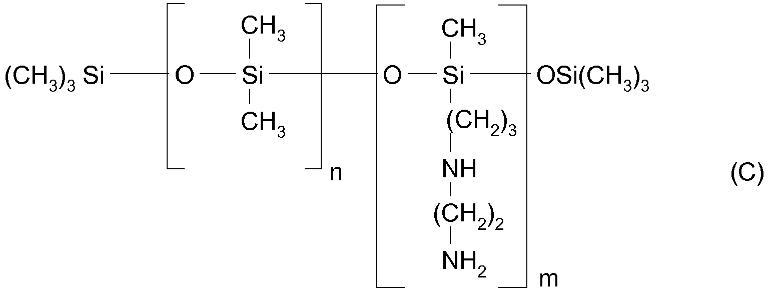 in which m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10.

According to a second embodiment, the amino silicones corresponding to formula (B) are chosen from the silicones of formula (D) below: 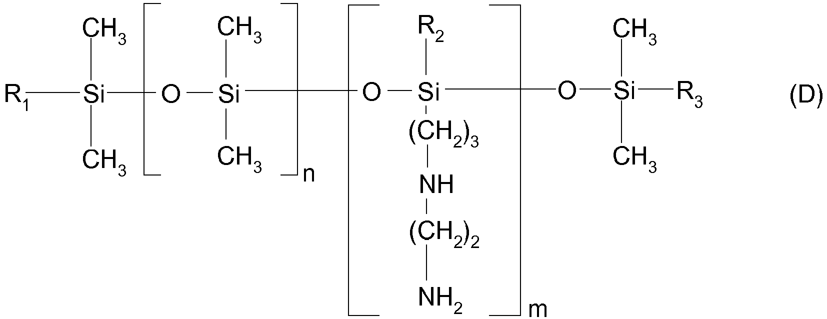 in which:
- m and n are numbers such that the sum (n + m) ranges from 1 to 1000 and in particular from 50 to 250 and more particularly from 100 to 200; it being possible for n to denote a number from 0 to 999 and in particular from 49 to 249 and more particularly from 125 to 175, and for m to denote a number from 1 to 1000 and in particular from 1 to 10, and more particularly from 1 to 5;
- R1, R2 and R3, which may be identical or different, represent a hydroxyl or C1-C4 alkoxy radical, at least one of the radicals R1 to R3 denoting an alkoxy radical.

Preferably, the alkoxy radical is a methoxy radical.

The hydroxy/alkoxy mole ratio preferably ranges from 0.2:1 to 0.4:1 and preferably from 0.25:1 to 0.35:1 and more particularly equals 0.3:1.

The weight-average molecular mass (Mw) of these silicones preferably ranges from 2000 to 1 000 000 and more particularly from 3500 to 200 000.

According to a third embodiment, the amino silicones corresponding to formula (B) are chosen from the silicones of formula (E) below: 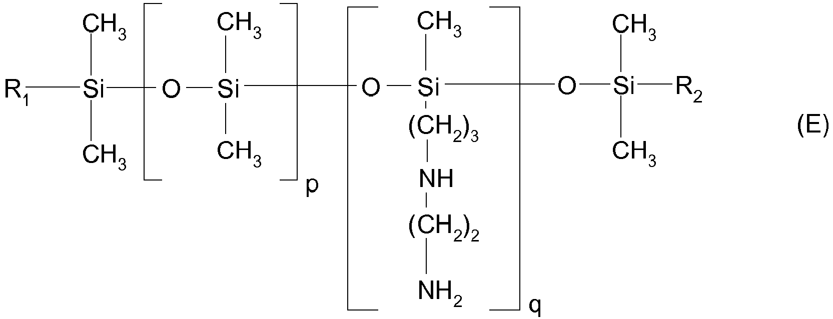 in which:
- p and q are numbers such that the sum (p + q) ranges from 1 to 1000, in particular from 50 to 350 and more particularly from 150 to 250; it being possible for p to denote a number from 0 to 999 and in particular from 49 to 349 and more particularly from 159 to 239, and for q to denote a number from 1 to 1000, in particular from 1 to 10 and more particularly from 1 to 5;
- R1 and R2, which are different, represent a hydroxyl or C₁-C₄ alkoxy radical, at least one of the radicals R1 or R2 denoting an alkoxy radical.

Preferably, the alkoxy radical is a methoxy radical.

The hydroxy/alkoxy mole ratio generally ranges from 1:0.8 to 1:1.1 and preferably from 1:0.9 to 1:1 and more particularly equals 1:0.95.

The weight-average molecular mass (Mw) of the silicone preferably ranges from 2000 to 200 000, even more particularly from 5000 to 100 000 and more particularly from 10 000 to 50 000.

The commercial products comprising silicones of structure (D) or (E) may include in their composition one or more other amino silicones of which the structure is different from formula (D) or (E).

A product containing amino silicones of structure (D) is sold by the company Wacker under the name Belsil^{®} ADM 652.

A product containing amino silicones of structure (E) is sold by Wacker under the name Fluid WR 1300^{®}.

When these amino silicones are used, one particularly advantageous embodiment consists in using them in the form of an oil-in-water emulsion. The oil-in-water emulsion may comprise one or more surfactants. The surfactants may be of any nature but are preferably cationic and/or nonionic. The numerical mean size of the silicone particles in the emulsion generally ranges from 3 nm to 500 nanometers. Preferably, in particular as amino silicones of formula (E), use is made of microemulsions of which the mean particle size ranges from 5 nm to 60 nanometers (limits included) and more particularly from 10 nm to 50 nanometers (limits included). Thus, use may be made according to the invention of the amino silicone microemulsions of formula (E) sold under the names Finish CT 96 E^{®} or SLM 28020^{®} by the company Wacker.

According to a fourth embodiment, the amino silicones corresponding to formula (B) are chosen from the silicones of formula (F) below: 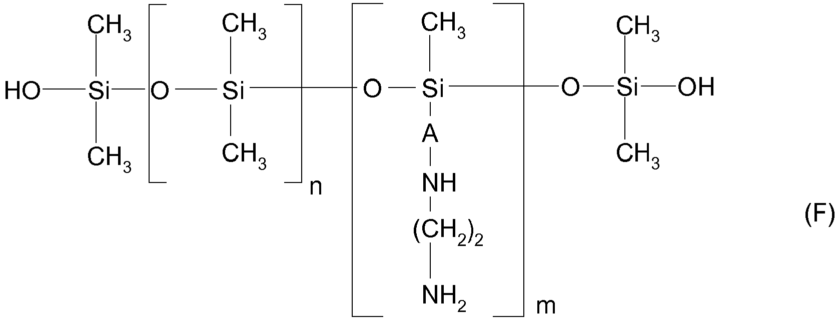 in which:
- m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
- A denotes a linear or branched alkylene radical containing from 4 to 8 carbon atoms and preferably 4 carbon atoms. This radical is preferably linear.

The weight-average molecular mass (Mw) of these amino silicones preferably ranges from 2000 to 1 000 000 and even more particularly from 3500 to 200 000.

A silicone corresponding to this formula is, for example, the Xiameter MEM 8299 Emulsion from Dow Corning.

According to a fifth embodiment, the amino silicones corresponding to formula (B) are chosen from the silicones of formula (G) below: 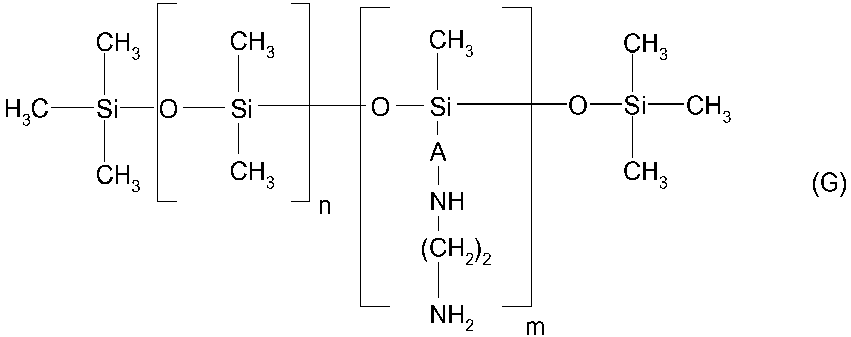 in which:
- m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10;
- A denotes a linear or branched alkylene radical containing from 4 to 8 carbon atoms and preferably 4 carbon atoms. This radical is preferably branched.

The weight-average molecular mass (Mw) of these amino silicones preferably ranges from 500 to 1 000 000 and even more particularly from 1000 to 200 000.

A silicone corresponding to this formula is, for example, DC2-8566 Amino Fluid from Dow Corning.
c) the amino silicones corresponding to formula (H): 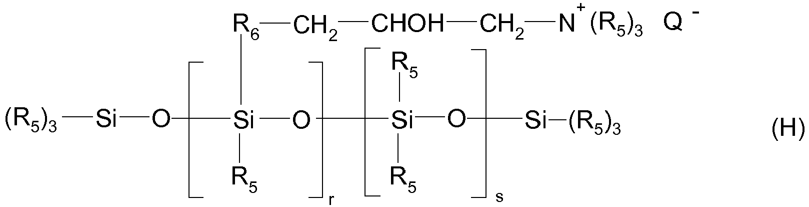 in which:
- R₅ represents a monovalent hydrocarbon-based radical containing from 1 to 18 carbon atoms, and in particular a C₁-C₁₈ alkyl or C₂-C₁₈ alkenyl, for example methyl, radical;
- R₆ represents a divalent hydrocarbon-based radical, in particular a C₁-C₁₈ alkylene radical or a divalent C₁-C₁₈, for example C₁-C₈, alkyleneoxy radical linked to the Si via an SiC bond;
- Q⁻ is an anion such as a halide, especially chloride, ion or an organic acid salt, especially acetate;
- r represents a mean statistical value ranging from 2 to 20 and in particular from 2 to 8;
- s represents a mean statistical value ranging from 20 to 200 and in particular from 20 to 50.

Such amino silicones are in particular described in patent US 4 185 087.
- d) the quaternary ammonium silicones of formula (I): 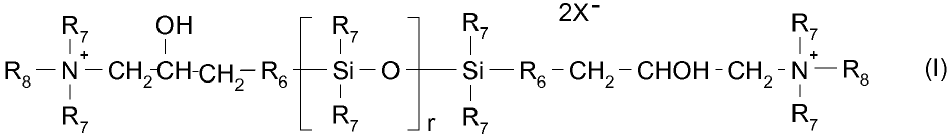 in which:
   - R₇, which may be identical or different, represent a monovalent hydrocarbon-based radical containing from 1 to 18 carbon atoms, and in particular a Ci-Cis alkyl radical, a C₂-C₁₈ alkenyl radical or a ring comprising 5 or 6 carbon atoms, for example a methyl radical;
   - R₆ represents a divalent hydrocarbon-based radical, in particular a C₁-C₁₈ alkylene radical or a divalent C₁-C₁₈, for example C₁-C₈, alkyleneoxy radical linked to the Si via an SiC bond;
   - R₈, which may be identical or different, represent a hydrogen atom, a monovalent hydrocarbon-based radical containing from 1 to 18 carbon atoms, and in particular a Ci-Cis alkyl radical, a C₂-C₁₈ alkenyl radical or a radical -R₆-NHCOR₇;
   - X⁻ is an anion such as a halide, especially chloride, ion or an organic acid salt, especially acetate;
   - r represents a mean statistical value ranging from 2 to 200 and in particular from 5 to 100.

These silicones are described, for example, in patent application EP-A 0 530 974.
e) the amino silicones of formula (J): 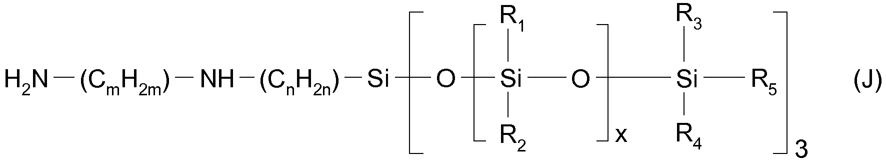 in which:
   - R₁, R₂, R₃ and R₄, which may be identical or different, denote a C₁-C₄ alkyl radical or a phenyl group,
   - R₅ denotes a C₁-C₄ alkyl radical or a hydroxyl group,
   - n is an integer ranging from 1 to 5,
   - m is an integer ranging from 1 to 5, and
   - x is chosen such that the amine number ranges from 0.01 to 1 meq/g.
f) the multiblock polyoxyalkylenated amino silicones, of the type (AB)n, A being a polysiloxane block and B being a polyoxyalkylenated block comprising at least one amine group.

Said silicones are preferably constituted of repeating units of the following general formulae:

[-(SiMe₂O)ₓSiMe₂ - R -N(R")- R'-O(C₂H₄O)ₐ(C₃H₆O)_{b} -R'-N(H)-R-]

or alternatively

[-(SiMe₂O)ₓSiMe₂ - R -N(R")- R'- O(C₂H₄O)ₐ(C₃H₆O)_{b}-]

in which:
- a is an integer greater than or equal to 1, preferably ranging from 5 to 200 and more particularly ranging from 10 to 100;
- b is an integer between 0 and 200, preferably ranging from 4 to 100 and more particularly between 5 and 30;
- x is an integer ranging from 1 to 10 000 and more particularly from 10 to 5000;
- R" is a hydrogen atom or a methyl;
- R, which may be identical or different, represent a divalent linear or branched C₂-C₁₂ hydrocarbon-based radical, optionally including one or more heteroatoms such as oxygen; preferably, R denotes an ethylene radical, a linear or branched propylene radical, a linear or branched butylene radical, or a -CH₂CH₂CH₂OCH₂CH(OH)CH₂- radical; preferentially R denotes a -CH₂CH₂CH₂OCH₂CH(OH)CH₂- radical;
- R', which may be identical or different, represent a linear or branched C₂-C₁₂ divalent hydrocarbon-based radical, optionally comprising one or more heteroatoms such as oxygen; preferably, R' denotes an ethylene radical, a linear or branched propylene radical, a linear or branched butylene radical, or a -CH₂CH₂CH₂OCH₂CH(OH)CH₂- radical; preferentially, R' denotes -CH(CH₃)-CH₂-.

The siloxane blocks preferably represent 50 mol% to 95 mol% of the total weight of the silicone, more particularly from 70 mol% to 85 mol%.

The amine content is preferably between 0.02 and 0.5 meq/g of copolymer in a 30% solution in dipropylene glycol, more particularly between 0.05 and 0.2.

The weight-average molecular mass (Mw) of the silicone is preferably between 5000 and 1 000 000 and more particularly between 10 000 and 200 000.

Mention may be made especially of the silicones sold under the names Silsoft A-843 or Silsoft A+ by Momentive.
g) and mixtures thereof.

Preferably, the composition contains at least one non-amino silicone of INCI name dimethicone and/or one amino silicone of INCI name amodimethicone.

The silicone(s) are present in a total amount of at least 0.1% by weight relative to the total weight of the composition, preferably at least 0.15%, more preferentially at least 0.3%, more preferably at least 0.5%, better still at least 0.75% and even better still 1% by weight relative to the total weight of the composition.

The silicone(s) are present in a total amount ranging from 0.1% to 15% by weight relative to the total weight of the composition, preferably from 0.15% to 10%, more preferentially from 0.3% to 7% by weight, more preferably from 0.5% to 5% by weight, better still from 0.75% to 4% by weight and even better still from 1% to 3% by weight relative to the total weight of the composition.

The total amount of amino silicone(s) ranges from 0.1% to 15% by weight, preferably from 0.1% to 10% by weight and better still from 0.15% to 5% by weight relative to the total weight of the composition.

Preferably, the weight ratio of the total amount of copolymer(s) of crotonic acid or crotonic acid derivative according to the invention to the total amount of silicone(s) ranges from 0.1 to 15, more preferentially from 0.5 to 10 and better still from 1 to 8.

The weight ratio of the total amount of copolymer(s) of crotonic acid or crotonic acid derivative according to the invention to the total amount of amino silicone(s) preferably ranges from 0.1 to 30, more preferentially from 0.5 to 25 and better still from 1 to 20.

### Pigments

The composition comprises one or more pigments.

The term "pigment" is understood to mean white or colored particles of any shape which are insoluble in the composition in which they are present.

The pigments that may be used are especially chosen from the organic and/or mineral pigments known in the art, especially those described in Kirk-Othmer's Encyclopedia of Chemical Technology and in Ullmann's Encyclopedia of Industrial Chemistry.

They may be natural, of natural origin, or not.

These pigments may be in pigment powder or paste form. They may be coated or uncoated.

The pigments may be chosen, for example, from mineral pigments, organic pigments, lakes, pigments with special effects, such as nacres or glitter flakes, and mixtures thereof.

The pigment may be a mineral pigment. The term "mineral pigment" means any pigment that satisfies the definition in Ullmann's encyclopedia in the chapter on inorganic pigments. Mention may be made, among mineral pigments of use in the present invention, of ochres, such as red ochre (clay (in particular kaolinite) and iron hydroxide (for example hematite)), brown ochre (clay (in particular kaolinite) and limonite) or yellow ochre (clay (in particular kaolinite) and goethite); titanium dioxide, optionally surface-treated; zirconium or cerium oxides; zinc, (black, yellow or red) iron or chromium oxides; manganese violet, ultramarine blue, chromium hydrate and ferric blue; or metal powders, such as aluminum powder or copper powder.

Mention may also be made of alkaline earth metal carbonates (such as calcium carbonate or magnesium carbonate), silicon dioxide, quartz and any other compound used as inert filler in cosmetic compositions, provided that these compounds contribute color or whiteness to the composition under the conditions under which they are employed.

The pigment may be an organic pigment. The term "organic pigment" means any pigment that satisfies the definition in Ullmann's encyclopedia in the chapter on organic pigments.

The organic pigment may especially be chosen from nitroso, nitro, azo, xanthene, pyrene, quinoline, anthraquinone, triphenylmethane, fluorane, phthalocyanine, metal-complex, isoindolinone, isoindoline, quinacridone, perinone, perylene, diketopyrrolopyrrole, indigo, thioindigo, dioxazine, triphenylmethane and quinophthalone compounds.

Use may also be made of any mineral or organic compound that is insoluble in the composition and standard in the cosmetics field, provided that these compounds give the composition color or whiteness under the conditions under which they are used, for example guanine, which, according to the refractive index of the composition, is a pigment.

In particular, the white or colored organic pigments may be chosen from carmine, carbon black, aniline black, azo yellow, quinacridone, phthalocyanine blue, the blue pigments codified in the Color Index under the references CI 42090, 69800, 69825, 73000, 74100, 74160, the yellow pigments codified in the Color Index under the references CI 11680, 11710, 15985, 19140, 20040, 21100, 21108, 47000, 47005, the green pigments codified in the Color Index under the references CI 61565, 61570, 74260, the orange pigments codified in the Color Index under the references CI 11725, 15510, 45370, 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, the pigments obtained by oxidative polymerization of indole or phenolic derivatives as described in patent FR 2 679 771.

Examples that may also be mentioned include pigmentary pastes of organic pigments, such as the products sold by the company Hoechst under the names:
- Cosmenyl Yellow I0G: Pigment Yellow 3 (CI 11710);
- Cosmenyl Yellow G: Pigment Yellow 1 (CI 11680);
- Cosmenyl Orange GR: Pigment Orange 43 (CI 71105);
- Cosmenyl Red R: Pigment Red 4 (CI 12085);
- Carmine Cosmenyl FB: Pigment Red 5 (CI 12490);
- Cosmenyl Violet RL: Pigment Violet 23 (CI 51319);
- Cosmenyl Blue A2R: Pigment Blue 15.1 (CI 74160);
- Cosmenyl Green GG: Pigment Green 7 (CI 74260);
- Cosmenyl Black R: Pigment Black 7 (CI 77266).

The pigments in accordance with the invention may also be in the form of composite pigments, as described in patent EP 1 184 426. These composite pigments may be composed especially of particles comprising a mineral core, at least one binder, which provides for the attachment of the organic pigments to the core, and at least one organic pigment which at least partially covers the core.

The organic pigment may also be a lake. The term "lake" means dyes adsorbed onto insoluble particles, the assembly thus obtained remaining insoluble during use.

The mineral substrates onto which the dyes are adsorbed are, for example, alumina, silica, calcium sodium borosilicate or calcium aluminum borosilicate and aluminum.

Among the dyes, mention may be made of carminic acid. Mention may also be made of the dyes known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090).

Mention may be made, as examples of lakes, of the product known under the following name: D&C Red 7 (CI 15 850:1).

The pigment may also be a pigment with special effects. The term "pigments with special effects" means pigments that generally create a colored appearance (characterized by a certain shade, a certain vivacity and a certain level of luminance) that is non-uniform and that changes as a function of the conditions of observation (light, temperature, angles of observation, etc.). They thus contrast with colored pigments that afford a standard uniform opaque, semitransparent or transparent shade.

Several types of pigment with special effects exist: those with a low refractive index, such as fluorescent or photochromic pigments, and those with a higher refractive index, such as nacres, interference pigments or glitter flakes.

Examples of pigments with special effects that may be mentioned include nacreous pigments such as mica coated with titanium or with bismuth oxychloride, colored nacreous pigments such as mica coated with titanium and with iron oxides, mica coated with iron oxide, mica coated with titanium and especially with ferric blue or with chromium oxide, mica coated with titanium and with an organic pigment of the abovementioned type, and also nacreous pigments based on bismuth oxychloride. Nacreous pigments that may be mentioned include the Cellini nacres sold by Engelhard (mica-TiO2-lake), Prestige sold by Eckart (mica-TiO2), Prestige Bronze sold by Eckart (mica-Fe2O3), and Colorona sold by Merck (mica-TiO2-Fe2O3).

Mention may also be made of the gold-colored nacres sold especially by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold especially by the company Merck under the name Bronze fine (17384) (Colorona) and Bronze (17353) (Colorona) and by the company Engelhard under the name Super bronze (Cloisonne); the orange nacres sold especially by the company Engelhard under the name Orange 363C (Cloisonne) and Orange MCR 101 (Cosmica) and by the company Merck under the name Passion orange (Colorona) and Matte orange (17449) (Microna); the brown nacres sold especially by the company Engelhard under the name Nu-antique copper 340XB (Cloisonne) and Brown CL4509 (Chromalite); the nacres with a coppery glint sold especially by the company Engelhard under the name Copper 340A (Timica); the nacres with a red glint sold especially by the company Merck under the name Sienna fine (17386) (Colorona); the nacres with a yellow glint sold especially by the company Engelhard under the name Yellow (4502) (Chromalite); the red nacres with a gold glint sold especially by the company Engelhard under the name Sunstone G012 (Gemtone); the pink nacres sold especially by the company Engelhard under the name Tan opale G005 (Gemtone); the black nacres with a gold glint sold especially by the company Engelhard under the name Nu antique bronze 240 AB (Timica), the blue nacres sold especially by the company Merck under the name Matte blue (17433) (Microna), the white nacres with a silvery glint sold especially by the company Merck under the name Xirona Silver, and the golden-green pink-orange nacres sold especially by the company Merck under the name Indian summer (Xirona), and mixtures thereof.

Still as examples of nacres, mention may also be made of particles comprising a borosilicate substrate coated with titanium oxide.

Particles comprising a glass substrate coated with titanium oxide are sold in particular under the name Metashine MC1080RY by the company Toyal.

Finally, examples of nacres that may also be mentioned include polyethylene terephthalate flakes, especially those sold by the company Meadowbrook Inventions under the name Silver 1P 0.004X0.004 (silver flakes).

It is also possible to envisage multilayer pigments based on synthetic substrates, such as alumina, silica, calcium sodium borosilicate, calcium aluminum borosilicate and aluminum.

The pigments with special effects may also be chosen from reflective particles, i.e. especially from particles whose size, structure, especially the thickness of the layer(s) of which they are made and their physical and chemical nature, and surface state, allow them to reflect incident light. This reflection may, where appropriate, have an intensity sufficient to create, at the surface of the composition or mixture, when the latter is applied to the substrate to be made up, highlight points that are visible to the naked eye, i.e. more luminous points which contrast with their surroundings by appearing to sparkle.

The reflective particles may be selected so as not to significantly alter the coloring effect generated by the coloring agents with which they are combined, and more particularly so as to optimize this effect in terms of color rendition. They may more particularly have a yellow, pink, red, bronze, orange, brown, gold and/or coppery color or glint.

These particles may have varied forms and may especially be in platelet or globular form, in particular in spherical form.

Irrespective of their form, the reflective particles may or may not have a multilayer structure, and, in the case of a multilayer structure, may have, for example, at least one layer of uniform thickness, especially of a reflective material.

When the reflective particles do not have a multilayer structure, they may be composed, for example, of metal oxides, especially titanium or iron oxides obtained synthetically.

When the reflective particles have a multilayer structure, they may comprise, for example, a natural or synthetic substrate, especially a synthetic substrate at least partially coated with at least one layer of a reflective material, especially of at least one metal or metallic material. The substrate may be made of one or more organic and/or mineral materials.

More particularly, it may be chosen from glasses, ceramics, graphite, metal oxides, aluminas, silicas, silicates, especially aluminosilicates and borosilicates, and synthetic mica, and mixtures thereof, this list not being limiting.

The reflective material may comprise a layer of metal or of a metallic material.

Reflective particles are described in particular in the documents

JP-A-09188830, JP-A-10158450, JP-A-10158541, JP-A-07258460 and JP-A-05017710.

Mention may also be made, still by way of example of reflective particles comprising a mineral substrate coated with a layer of metal, of the particles comprising a borosilicate substrate coated with silver.

Particles comprising a glass substrate coated with silver, in the form of platelets, are sold under the name Microglass Metashine REFSX 2025 PS by Toyal. Particles with a glass substrate coated with nickel/chromium/molybdenum alloy are sold under the name Crystal Star GF 550 and GF 2525 by this same company.

Use may also be made of particles comprising a metal substrate, such as silver, aluminum, iron, chromium, nickel, molybdenum, gold, copper, zinc, tin, magnesium, steel, bronze or titanium, said substrate being coated with at least one layer of at least one metal oxide, such as titanium oxide, aluminum oxide, iron oxide, cerium oxide, chromium oxide, silicon oxides and mixtures thereof.

Examples that may be mentioned include aluminum powder, bronze powder or copper powder coated with SiO2 sold under the name Visionaire by the company Eckart.

Mention may also be made of pigments with an interference effect which are not attached to a substrate, such as liquid crystals (Helicones HC from Wacker) or interference holographic glitter flakes (Geometric Pigments or Spectra f/x from Spectratek). Pigments with special effects also comprise fluorescent pigments, whether these are substances that are fluorescent in daylight or that produce an ultraviolet fluorescence, phosphorescent pigments, photochromic pigments, thermochromic pigments and quantum dots, sold, for example, by the company Quantum Dots Corporation.

Quantum dots are luminescent semiconductive nanoparticles capable of emitting, under light excitation, irradiation with a wavelength of between 400 nm and 700 nm. These nanoparticles are known from the literature. In particular, they may be synthesized according to the processes described, for example, in US 6 225 198 or US 5 990 479, in the publications cited therein and also in the following publications: Dabboussi B.O. et al., "(CdSe)ZnS core-shell quantum dots: synthesis and characterisation of a size series of highly luminescent nanocrystallites", Journal of Physical Chemistry B, vol. 101, 1997, pp. 9463-9475, and Peng, Xiaogang et al., "Epitaxial growth of highly luminescent CdSe/CdS core/shell nanocrystals with photostability and electronic accessibility", Journal of the American Chemical Society, vol. 119, No. 30, pp. 7019-7029.

The variety of pigments that may be used in the present invention makes it possible to obtain a wide range of colors, and also particular optical effects such as metallic effects or interference effects.

The size of the pigment used in the cosmetic composition according to the present invention is generally between 10 nm and 200 µm, preferably between 20 nm and 80 µm and more preferably between 30 nm and 50 µm.

The pigments may be dispersed in the product by means of a dispersant.

The dispersant serves to protect the dispersed particles against their agglomeration or flocculation. This dispersant may be a surfactant, an oligomer, a polymer or a mixture of several thereof, bearing one or more functionalities with strong affinity for the surface of the particles to be dispersed. In particular, they may become physically or chemically attached to the surface of the pigments. These dispersants also contain at least one functional group that is compatible with or soluble in the continuous medium. In particular, 12-hydroxystearic acid esters in particular and C8 to C20 fatty acid esters of polyols such as glycerol or diglycerol are used, such as poly(12-hydroxystearic acid) stearate with a molecular weight of about 750 g/mol, such as the product sold under the name Solsperse 21 000 by the company Avecia, polyglyceryl-2 dipolyhydroxystearate (CTFA name) sold under the reference Dehymyls PGPH by the company Henkel, or polyhydroxystearic acid such as the product sold under the reference Arlacel P100 by the company Uniqema, and mixtures thereof.

As other dispersants that may be used in the compositions of the invention, mention may be made of quaternary ammonium derivatives of polycondensed fatty acids, for instance Solsperse 17 000 sold by the company Avecia, and polydimethylsiloxane/oxypropylene mixtures such as those sold by the company Dow Corning under the references DC2-5185 and DC2-5225 C.

The pigments used in the cosmetic composition according to the invention may be surface-treated with an organic agent.

Thus, the pigments that have been surface-treated beforehand, which are useful in the context of the invention, are pigments that have totally or partially undergone a surface treatment of chemical, electronic, electrochemical, mechanochemical or mechanical nature, with an organic agent such as those described especially in Cosmetics and Toiletries, February 1990, Vol. 105, pages 53-64, before being dispersed in the composition in accordance with the invention. These organic agents may be chosen, for example, from waxes, for example carnauba wax and beeswax; fatty acids, fatty alcohols and derivatives thereof, such as stearic acid, hydroxystearic acid, stearyl alcohol, hydroxystearyl alcohol and lauric acid and derivatives thereof; anionic surfactants; lecithins; sodium, potassium, magnesium, iron, titanium, zinc or aluminum salts of fatty acids, for example aluminum stearate or laurate; metal alkoxides; polyethylene; (meth)acrylic polymers, for example polymethyl methacrylates; polymers and copolymers containing acrylate units; alkanolamines; silicone compounds, for example silicones, polydimethylsiloxanes; organofluorine compounds, for example perfluoroalkyl ethers; fluorosilicone compounds.

The surface-treated pigments that are useful in the cosmetic composition according to the invention may also have been treated with a mixture of these compounds and/or may have undergone several surface treatments.

The surface-treated pigments that are useful in the context of the present invention may be prepared according to surface-treatment techniques that are well known to those skilled in the art, or may be commercially available in the required form.

Preferably, the surface-treated pigments are coated with an organic layer.

The organic agent with which the pigments are treated may be deposited on the pigments by evaporation of solvent, chemical reaction between the molecules of the surface agent or creation of a covalent bond between the surface agent and the pigments.

The surface treatment may thus be performed, for example, by chemical reaction of a surface agent with the surface of the pigments and creation of a covalent bond between the surface agent and the pigments or the fillers. This method is especially described in patent US 4 578 266.

An organic agent covalently bonded to the pigments will preferably be used.

The agent for the surface treatment may represent from 0.1% to 50% by weight, preferably from 0.5% to 30% by weight and even more preferentially from 1% to 10% by weight relative to the total weight of the surface-treated pigment.

Preferably, the surface treatments of the pigments are chosen from the following treatments:
- a PEG-silicone treatment, for instance the AQ surface treatment sold by LCW;
- a methicone treatment, for instance the SI surface treatment sold by LCW;
- a dimethicone treatment, for instance the Covasil 3.05 surface treatment sold by LCW;
- a dimethicone/trimethyl siloxysilicate treatment, for instance the Covasil 4.05 surface treatment sold by LCW;
- a magnesium myristate treatment, for instance the MM surface treatment sold by LCW;
- an aluminum dimyristate treatment, such as the MI surface treatment sold by Miyoshi;
- a perfluoropolymethylisopropyl ether treatment, for instance the FHC surface treatment sold by LCW;
- an isostearyl sebacate treatment, for instance the HS surface treatment sold by Miyoshi;
- a perfluoroalkyl phosphate treatment, for instance the PF surface treatment sold by Daito;
- an acrylate/dimethicone copolymer and perfluoroalkyl phosphate treatment, for instance the FSA surface treatment sold by Daito;
- a polymethylhydrogenosiloxane/perfluoroalkyl phosphate treatment, for instance the FS01 surface treatment sold by Daito;
- an acrylate/dimethicone copolymer treatment, for instance the ASC surface treatment sold by Daito;
- an isopropyl titanium triisostearate treatment, for instance the ITT surface treatment sold by Daito;
- an acrylate copolymer treatment, for instance the APD surface treatment sold by Daito;
- a perfluoroalkyl phosphate/isopropyl titanium triisostearate treatment, for instance the PF + ITT surface treatment sold by Daito.

Preferably, the pigment is chosen from mineral or mixed mineral-organic pigments.

The amount of pigment(s) may range from 0.01% to 30% by weight, more particularly from 0.05% to 20% by weight, preferably from 0.1% to 15% by weight and preferably from 1% to 10% by weight relative to the total weight of the composition.

The composition of the invention may contain colored or coloring species other than the pigments according to the invention, such as direct dyes or dye precursors.

### Thickener

According to a preferred embodiment, the composition according to the invention comprises at least one thickener, preferably chosen from natural polymers, carboxyvinyl polymers such as homopolymers or copolymers of acrylic and/or methacrylic acid and/or ester, which are preferably crosslinked, crosslinked thickening polyacrylamides and associative polymers comprising at least one hydrophilic unit and at least one fatty chain.

According to the present invention, the term "thickener" refers to a compound which, by its presence at a concentration of 0.05% by weight, increases the viscosity of a composition into which it is introduced by at least 20 mPa.s (cps) preferably by at least 50 mPa.s (cps) at room temperature

(25°C), at atmospheric pressure and at a shear rate of 1 s⁻¹ (the viscosity may be measured using a cone/plate viscometer, a Haake R600 rheometer or the like).

The thickener(s) may be chosen especially from carboxyvinyl polymers such as crosslinked acrylic acid homopolymers (carbomer) such as those sold under the name Carbopol by the company Goodrich, polyacrylates and polymethacrylates such as the products sold under the names Lubrajel or Norgel by the company Guardian or under the name Hispagel by the company Hispano Chimica; polyacrylamides such as the product sold under the name Sepigel 305 by the company SEPPIC; polysaccharides such as alginates, cellulose and derivatives thereof, especially carboxymethylcellulose, hydroxymethylcellulose, hydroxypropylcellulose and microcrystalline cellulose; natural gums such as xanthan gum, guar gum, locust bean gum, acacia gum, scleroglucans, chitin and chitosan derivatives, carrageenans; or clays such as montmorillonite, bentones and magnesium aluminum silicates (Veegum).

According to a particular embodiment of the invention, the composition comprises at least one thickener chosen from crosslinked acrylic and/or methacrylic acid polymers.

According to a particular embodiment of the invention, the composition comprises at least one thickener chosen from crosslinked acrylic acid homopolymers (INCI name Carbomer).

The thickener may be present in the composition in a total content ranging from 0.01% to 10% by weight relative to the weight of the composition, preferably from 0.1% to 5% by weight relative to the weight of the composition, preferably from 0.4% to 2% by weight relative to the weight of the composition.

The composition according to the invention advantageously comprises water, which may preferably be present in a content ranging from 20% to 98% by weight relative to the weight of the composition.

### Additives

The compositions may also comprise at least one agent commonly used in cosmetics, for example chosen from reducing agents, fatty substances other than silicones, organic solvents, softeners, antifoams, moisturizers, UV-screening agents, peptizers, solubilizers, fragrances, anionic, cationic, nonionic or amphoteric surfactants, proteins and vitamins.

The above additives are generally present in an amount for each of them of between 0.01% and 20% by weight relative to the weight of the composition.

Needless to say, a person skilled in the art will take care to select this or these optional additive(s) such that the advantageous properties intrinsically associated with the formation of the coating in accordance with the invention are not, or are not substantially, adversely affected.

### Presentation form

The composition according to the invention may especially be in the form of a suspension, a dispersion, a gel, an emulsion, especially an oil-in-water (O/W) or water-in-oil (W/O) emulsion, or a multiple emulsion (W/O/W or polyol/O/W or O/W/O), in the form of a cream, a mousse, a stick, a dispersion of vesicles, especially of ionic or nonionic lipids, or a two-phase or multiphase lotion. Preferably, the composition is in the form of a gel.

A person skilled in the art may select the appropriate presentation form, and also the method for preparing it, on the basis of his general knowledge, taking into account first the nature of the constituents used, especially their solubility in the support, and secondly the application envisaged for the composition.

### Organic solvents

The composition according to the invention may comprise one or more organic solvents.

Examples of organic solvents that may be mentioned include lower C₁-C₄ alkanols, such as ethanol and isopropanol; polyols and polyol ethers, for instance 2-butoxyethanol, propylene glycol, propylene glycol monomethyl ether and diethylene glycol monoethyl ether and monomethyl ether, and also aromatic alcohols, for instance benzyl alcohol or phenoxyethanol, and mixtures thereof.

Preferably, the composition according to the invention comprises one or more organic solvents.

When they are present, the organic solvents are present in proportions preferably inclusively between 0.1% and 40% by weight approximately relative to the total weight of the dye composition, more preferentially between 1% and 30% by weight approximately and even more particularly inclusively between 5% and 25% by weight relative to the total weight of the composition.

### Process

The composition described above may be used on wet or dry keratin fibers, and also on any type of fair or dark, natural or dyed, permanent-waved, bleached or relaxed keratin fibers.

The application to the fibers may be performed via any standard means, in particular using a comb, a fine brush, a coarse brush or with the fingers.

Preferably, if the fibers are dried, they are dried, in addition to a supply of heat, with a flow of air.

During drying, a mechanical action on the locks may be exerted, such as combing, brushing or passing the fingers through. This operation may similarly be performed once the fibers have been dried, naturally or otherwise.

The drying step of the process of the invention may be performed with a hood, a hair dryer, a straightening iron, a Climazon, etc.

When the drying step is performed with a hood or a hairdryer, the drying temperature is between 30 and 110°C and preferably between 50 and 90°C.

When the drying step is performed with a straightening iron, the drying temperature is between 110 and 220°C and preferably between 140 and 200°C.

### EXAMPLES

### Example 1:

### Compositions (g AM/100 g)

| | **A** |
|---|---|
| **VA/crotonates/vinyl neodecanoate copolymer** | **3** |
| **Carbomer** | **0.75** |
| **Dimethicone** | **0.8** |
| **Amodimethicone** | **0.2** |
| **Synthetic mica and titanium dioxide and Red 7 calcium lake on barium sulfate substrate** | **7** |
| **Neutralizers** | **qs** |
| **Preserving agent, fragrance** | **qs** |
| **Ethanol** | **7.5** |
| **PEG-40 hydrogenated castor oil** | **1** |
| **Water** | **qs 100** |

### Protocol

Composition A is applied to locks of yak hair at a rate of 1 g of composition per gram of lock. The locks are then combed, dried with a hairdryer and then combed again.

### Results: "cosmetic feel" performance

The performance qualities in terms of cosmetic feel were evaluated on dried locks by five experts, in a blind test.

In 100% of the cases, the experts judged that composition A according to the invention afforded smooth locks with clearly individualized hair strands, having a pleasant cosmetic feel, especially good softness, good suppleness and absence of tackiness.

### Example 2:

### Compositions (g AM/100 g)

| | **A1 Invention** | **B1 comparative** | **C1 comparative** |
|---|---|---|---|
| **Phenoxyethanol** | **0.7** | **0.7** | **0.7** |
| **Amodimethicone** | **0.2** | **0.2** | **0.2** |
| **Dimethicone** | **0.8** | **0.8** | **0.8** |
| **CI 77891 (and) synthetic fluorophlogopite (and) CI 15850** | **10** | **10** | **10** |
| **VA/crotonates/vinyl neodecanoate copolymer** | **3** | **-** | **-** |
| **Carbomer** | **0.75** | **0.75** | **0.75** |
| **Polyvinylcaprolactam** | **-** | **3** | **-** |
| **VP/dimethylaminoethyl methacrylate copolymer** | **-** | **-** | **3** |
| **Ethanol** | **7.5** | **7.5** | **7.5** |
| **Water** | **qs 100** | **qs 100** | **qs 100** |
| **PEG-40 Hydrogenated castor oil** | **1** | **1** | **1** |

### Protocol

Composition A1, B1 or C1 is applied to locks of yak hair at a rate of 1 g of composition per gram of lock.

The locks are dried with a hairdryer and then combed.

The locks are then rubbed on a white cloth.

### Results: "transfer-resistance" performance

The performance qualities in terms of transfer resistance were evaluated by five experts, in a blind test, who visually evaluated the amount of pigment present on the white cloth after rubbing.

In 100% of the cases, the experts judged that composition A1 according to the invention, compared with compositions B1 and C1, led to a very markedly smaller amount of pigment present on the cloth than the amount deposited by compositions B1 and C1. Composition A1 according to the invention thus has better transfer-resistance properties than the comparative compositions B1 and C1.

### Example 3:

### Compositions (g AM/100g)

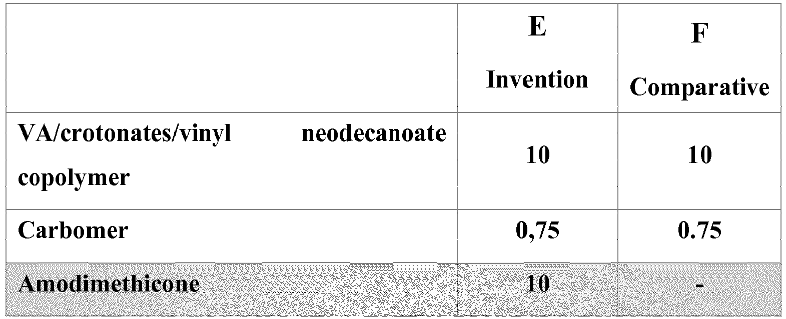

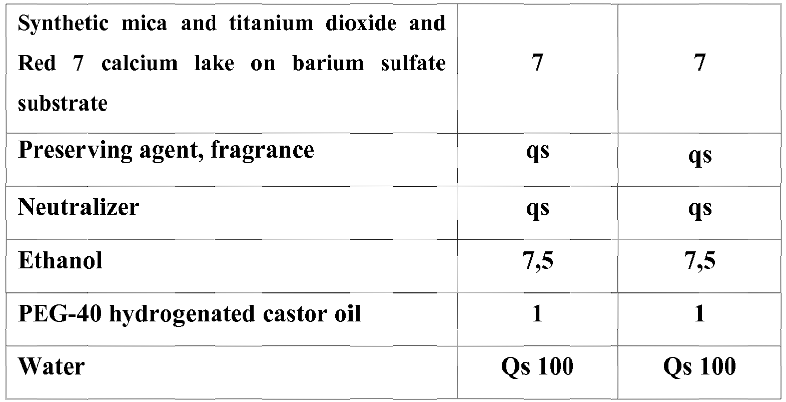

### Protocol:

The compositions E and F are applied on the locks of Yak hair at a rate of 1g of composition applied per gram of locks of hair.

After application, the locks of hair are combed, dried with a hair-drier and then combed again.

### Results: Suppleness, natural touch and individualization of the hair

In order to evaluate the suppleness and the individualization of the hair, the locks of hair are fixed at one extremity of the lock and are maintained horizontal at the other extremity of the lock. The non-fixed end of the lock is then released and the behaviour of the locks is observed:
The more the locks of the hair remains horizontal, the more the locks of hair will be rigid and agglomerated into packs (not individualized)

The more the locks of hair fall with a "curtain" effect (not agglomerated into packs), the more the locks of hair will be individualized, loose and suppleness.

A picture of the locks of hair has been taken 10 seconds after releasing the locks. The results obtained are disclosed herewith:

### Comparative example F on the picture (see Fig.1):

The lock stays horizontal. The lock of hair is agglomerated into packs, not individualized and with not good suppleness.

Moreover, the touch is rigid and not natural.

### Composition of the invention E on the picture (see Fig.1):

The lock of hair falls with a "curtain" effect, the lock of hair is well individualized.

Moreover, the touch is suppleness and natural.

The lock of hair treated with composition E according to the invention has a more natural behaviour, the hairs are well individualized and have a good suppleness compared to the lock of hair treated with comparative composition F.

### Example 4:

### Compositions (g AM/100g)

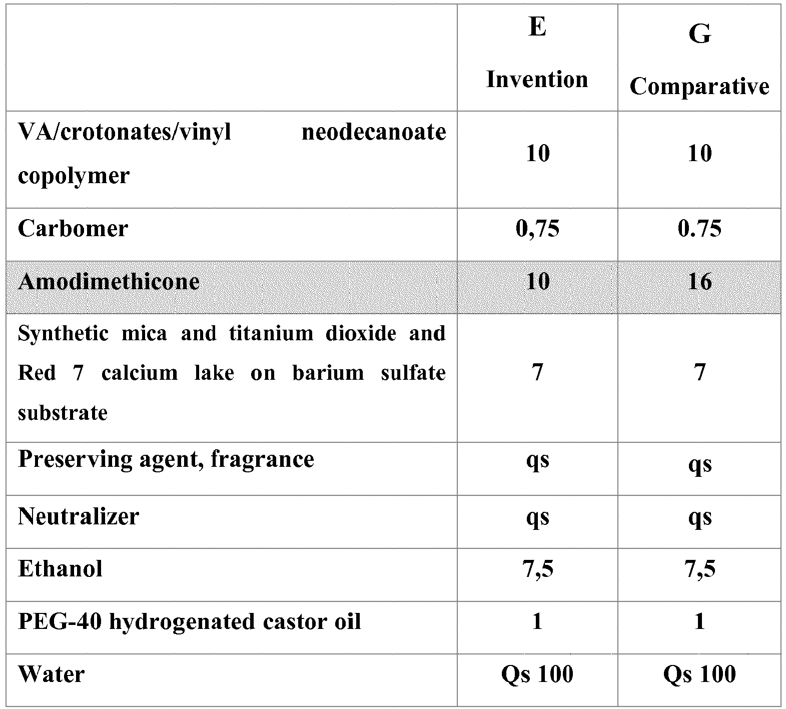

### Protocol:

The compositions E and G are applied on the locks of Yak hair at a rate of 1g of composition applied per gram of locks of hair.

After application, the locks of hair are combed, dried with a hair-drier and then combed again.

### Results: Suppleness, natural touch and individualization of the hair

In order to evaluate the suppleness and the individualization of the hair, the locks of hair are fixed at one extremity of the lock and are maintained horizontal at the other extremity of the lock. The non-fixed end of the lock is then released and the behaviour of the locks is observed:
The more the locks of the hair remains horizontal, the more the locks of hair will be rigid and agglomerated into packs (not individualized)

The more the locks of hair fall with a "curtain" effect (not agglomerated into packs), the more the locks of hair will be individualized, loose and suppleness.

A picture of the locks of hair has been taken 10 seconds after releasing the locks. The results obtained are disclosed herewith:

### Comparative example G on the picture (see Fig. 2):

The lock stays horizontal. The lock of hair is agglomerated into packs, not individualized and with not good suppleness.

Moreover, the touch is rigid and not natural.

### Composition of the invention E on the picture (see Fig.2):

The lock of hair falls with a "curtain" effect, the lock of hair is well individualized.

Moreover, the touch is suppleness and natural.

The lock of hair treated with composition E according to the invention has a more natural behaviour, the hairs are well individualized and have a good suppleness compared to the lock of hair treated with comparative composition G.

## Claims

1. Process for dyeing keratin fibers, in particular human keratin fibers such as the hair, comprising the application to said fibers of a composition comprising (i) one or more copolymers derived from the polymerization of at least one crotonic acid monomer or crotonic acid derivative and of at least one vinyl ester monomer, (ii) one or more amino silicones, the total amount of amino silicone(s) ranging from 0.1% to 15% by weight relative to the total weight of the composition, and (iii) one or more pigments; and wherein the total amount of silicone(s) ranges from 0.1% to 15% by weight relative to the total weight of the composition.

2. The process as claimed in claim 1, **characterized in that** said copolymer(s) are derived from the polymerization of at least one crotonic acid monomer and of at least one vinyl ester monomer.

3. The process as claimed in either of the preceding claims, **characterized in that** said at least one crotonic acid derivative is chosen from crotonic acid esters or amides.

4. The process as claimed in any one of the preceding claims, **characterized in that** said at least one crotonic acid derivative is chosen from the crotonic acid esters of formula CH₃CH=CHCOOR'1 with R'1 representing a linear, branched or cyclic, saturated or unsaturated, optionally aromatic (aryl, aralkyl or alkylaryl) carbon-based and especially hydrocarbon-based (alkyl) chain, containing 1 to 30 carbon atoms, optionally comprising one or more functions chosen from -OH, -OR' with R' C1-C6 alkyl (alkoxy), -CN, -X (halogen, especially Cl, F, Br or I) such as methyl crotonoate and ethyl crotonoate.

5. The process as claimed in any one of the preceding claims, **characterized in that** said at least one crotonic acid derivative is chosen from the crotonic acid amides of formula CH₃CH=CHCONR'2R"2 with R'2 and R"2, which may be identical or different, representing hydrogen or a linear, branched or cyclic, saturated or unsaturated, optionally aromatic, carbon-based and especially hydrocarbon-based (alkyl) chain, containing 1 to 30 carbon atoms, optionally comprising one or more functions chosen from -OH, -OR' with R' C1-C6 alkyl (alkoxy), -CN, -X (halogen, especially Cl, F, Br or I).

6. The process as claimed in claims 1 to 5, **characterized in that** said copolymer(s) are derived from the polymerization of at least one crotonic acid monomer or crotonic acid derivative and of at least two different vinyl ester monomers.

7. The process as claimed in claims 1 to 6, **characterized in that** said vinyl ester monomer(s) are chosen from vinyl acetate, vinyl propionate, vinyl butyrate (or butanoate), vinyl ethylhexanoate, vinyl neononanoate, vinyl neododecanoate, vinyl neodecanoate, vinyl pivalate, vinyl cyclohexanoate, vinyl benzoate, vinyl 4-tert-butylbenzoate and vinyl trifluoroacetate, preferably from vinyl acetate, vinyl propionate and vinyl neodecanoate, better still from vinyl acetate and vinyl neodecanoate.

8. The process as claimed in claims 1 to 7, **characterized in that** said copolymer(s) are chosen from copolymers derived from the polymerization of crotonic acid, vinyl acetate and vinyl propionate, copolymers derived from the polymerization of crotonic acid, vinyl acetate and vinyl neodecanoate, and mixtures thereof.

9. The process as claimed in claims 1 to 8, **characterized in that** the copolymer is a crotonic acid/vinyl acetate/vinyl neodecanoate terpolymer.

10. The process as claimed in claims 1 to 9, **characterized in that** said copolymers also comprise other monomers such as allylic or methallylic esters or vinyl ethers.

11. The process as claimed in any one of the preceding claims, in which said crotonic acid copolymer derived from the polymerization of at least one crotonic acid monomer or crotonic acid derivative and of at least one vinyl ester monomer is present in an amount ranging from 0.05% to 15% by weight relative to the weight of the composition, preferably from 0.1% to 10% by weight relative to the weight of the composition, preferably from 1% to 5% by weight relative to the total weight of the composition.

12. The process as claimed in any one of the preceding claims, **characterized in that** it contains at least two different silicones.

13. The process as claimed in the preceding claim, **characterized in that** it contains at least one silicone whose INCI name is dimethicone.

14. The process as claimed in any one of the preceding claims, **characterized in that** said amino silicone has the INCI name amodimethicone.

15. The process as claimed in any one of the preceding claims, **characterized in that** said silicone(s) are present in a total amount of at least 0.15%, more preferentially of at least 0.3%, more preferably of at least 0.5%, better still of at least 0.75% and even better still 1% by weight relative to the total weight of the composition.

16. The process as claimed in any one of the preceding claims, **characterized in that** said silicone(s) are present in a total amount ranging from 0.15% to 10%, more preferentially from 0.3% to 7% by weight, more preferably from 0.5% to 5% by weight, better still from 0.75% to 4% by weight and even better still from 1% to 3% by weight relative to the total weight of the composition.

17. The process as claimed in any one of the preceding claims, **characterized in that** the weight ratio of the total amount of copolymer(s) of crotonic acid or crotonic acid derivative to the total amount of silicone(s) ranges from 0.1 to 15, more preferentially from 0.5 to 10 and better still from 1 to 8.

18. The process as claimed in any one of the preceding claims, **characterized in that** it also comprises a thickener, preferably chosen from crosslinked copolymers of acrylic and/or methacrylic acid, preferably from crosslinked acrylic acid homopolymers.

19. A cosmetic composition comprising (i) a crotonic acid/vinyl acetate/vinyl neodecanoate terpolymer, (ii) one or more amino silicones, the total amount of amino silicone ranging from 0.1% to 15% by weight relative to the total weight of the composition, and (iii) one or more pigments; and wherein the total amount of silicone(s) ranges from 0.1% to 15% by weight relative to the total weight of the composition.

20. The use of the composition according to claim 19, for the dyeing of keratin fibers, in particular human keratin fibers such as the hair.

## Patentansprüche

1. Verfahren zum Färben von Keratinfasern, insbesondere menschlichen Keratinfasern wie dem Haar, bei dem man auf die Fasern eine Zusammensetzung aufbringt, die (i) ein oder mehrere Copolymere, die aus der Polymerisation von mindestens einem Crotonsäure-Monomer oder Crotonsäure-Derivat und mindestens einem Vinylester-Monomer stammen, (ii) ein oder mehrere Aminosilikone, wobei die Gesamtmenge an Aminosilikon bzw. Aminosilikonen im Bereich von 0,1 bis 15 Gew.-%, bezogen auf das Gesamtgewicht der Zusammensetzung, liegt, und (iii) ein oder mehrere Pigmente umfasst; und wobei die Gesamtmenge an Silikon bzw. Silikonen im Bereich von 0,1 bis 15 Gew.-%, bezogen auf das Gesamtgewicht der Zusammensetzung, liegt.

2. Zusammensetzung nach Anspruch 1, **dadurch gekennzeichnet, dass** das Copolymer bzw. die Copolymere aus der Polymerisation von mindestens einem Crotonsäure-Monomer und mindestens einem Vinylester-Monomer stammen.

3. Verfahren nach einem der vorhergehenden Ansprüche, **dadurch gekennzeichnet, dass** das mindestens eine Crotonsäure-Derivat aus Crotonsäureestern oder - amiden ausgewählt ist.

4. Verfahren nach einem der vorhergehenden Ansprüche, **dadurch gekennzeichnet, dass** das mindestens eine Crotonsäure-Derivat aus den Crotonsäureestern der Formel CH₃CH=CHCOOR'1, wobei R'1 für eine lineare, verzweigte oder cyclische, gesättigte oder ungesättigte, gegebenenfalls aromatische kohlenstoffbasierte Kette (Aryl-, Aralkyl- oder Alkylarylkette) und insbesondere kohlenwasserstoffbasierte Kette (Alkylkette) mit 1 bis 30 Kohlenstoffatomen, die gegebenenfalls eine oder mehrere Funktionen, die aus -OH, -OR', wobei R' für C1-C6-Alkyl steht (Alkoxy), -CN, -X (Halogen, insbesondere Cl, F, Br oder I), ausgewählt sind, umfasst, steht, wie Crotonsäuremethylester und Crotonsäureethylester, ausgewählt ist.

5. Verfahren nach einem der vorhergehenden Ansprüche, **dadurch gekennzeichnet, dass** das mindestens eine Crotonsäure-Derivat aus den Crotonsäureamiden der Formel CH₃CH=CHCONR'2R"2, wobei R'2 und R"2, die gleich oder verschieden sein können, für Wasserstoff oder eine lineare, verzweigte oder cyclische, gesättigte oder ungesättigte, gegebenenfalls aromatische kohlenstoffbasierte insbesondere kohlenwasserstoffbasierte Kette (Alkylkette) mit 1 bis 30 Kohlenstoffatomen, die gegebenenfalls eine oder mehrere Funktionen, die aus -OH, -OR', wobei R' für C1-C6-Alkyl steht (Alkoxy), -CN, -X (Halogen, insbesondere Cl, F, Br oder I), ausgewählt sind, umfasst, stehen, ausgewählt ist.

6. Verfahren nach den Ansprüchen 1 bis 5, **dadurch gekennzeichnet, dass** das Copolymer bzw. die Copolymere aus der Polymerisation von mindestens einem Crotonsäure-Monomer oder Crotonsäure-Derivat und mindestens zwei verschiedenen Vinylester-Monomeren stammt bzw. stammen.

7. Verfahren nach den Ansprüchen 1 bis 6, **dadurch gekennzeichnet, dass** das Vinylester-Monomer bzw. die Vinylester-Monomere aus Vinylacetat, Vinylpropionat, Vinylbutyrat (oder Vinylbutanoat), Vinylethylhexanoat, Vinylneononanoat, Vinylneododecanoat, Vinylneodecanoat, Vinylpivalat, Vinylcyclohexanoat, Vinylbenzoat, Vinyl-4-tert-butylbenzoat und Vinyltrifluoracetat, vorzugsweise aus Vinylacetat, Vinylpropionat und Vinylneodecanoat, noch besser aus Vinylacetat und Vinylneodecanoat, ausgewählt ist bzw. sind.

8. Verfahren nach den Ansprüchen 1 bis 7, **dadurch gekennzeichnet, dass** das Copolymer bzw. die Copolymere aus Copolymeren, die aus der Polymerisation von Crotonsäure, Vinylacetat und Vinylpropionat stammen, Copolymeren, die aus der Polymerisation von Crotonsäure, Vinylacetat und Vinylneodecanoat stammen, und Mischungen davon ausgewählt ist bzw. sind.

9. Verfahren nach den Ansprüchen 1 bis 8, **dadurch gekennzeichnet, dass** es sich bei dem Copolymer um ein Crotonsäure/Vinylacetat/Vinylneodecanoat-Terpolymer handelt.

10. Verfahren nach den Ansprüchen 1 bis 9, **dadurch gekennzeichnet, dass** die Copolymere außerdem andere Monomere wie Allyl- oder Metallylester oder Vinylether umfassen.

11. Verfahren nach einem der vorhergehenden Ansprüche, wobei das Crotonsäure-Copolymer, das aus der Polymerisation von mindestens einem Crotonsäure-Monomer oder Crotonsäure-Derivat und mindestens einem Vinylester-Monomer stammt, in einer Menge im Bereich von 0,05 bis 15 Gew.-%, bezogen auf das Gewicht der Zusammensetzung, vorzugsweise von 0,1 bis 10 Gew.-%, bezogen auf das Gewicht der Zusammensetzung, vorzugsweise von 1 bis 5 Gew.-%, bezogen auf das Gesamtgewicht der Zusammensetzung, vorliegt.

12. Verfahren nach einem der vorhergehenden Ansprüche, **dadurch gekennzeichnet, dass** sie mindestens zwei verschiedene Silikone enthält.

13. Verfahren nach dem vorhergehenden Anspruch, **dadurch gekennzeichnet, dass** sie mindestens ein Silikon enthält, dessen INCI-Name Dimethicone lautet.

14. Verfahren nach einem der vorhergehenden Ansprüche, **dadurch gekennzeichnet, dass** das Aminosilikon den INCI-Namen Amodimethicone hat.

15. Verfahren nach einem der vorhergehenden Ansprüche, **dadurch gekennzeichnet, dass** das Silikon bzw. die Silikone in einer Gesamtmenge von mindestens 0,15 Gew.-%, weiter bevorzugt mindestens 0,3 Gew.-%, weiter bevorzugt mindestens 0,5 Gew.-%, noch besser mindestens 0,75 Gew.-% und sogar noch besser 1 Gew.-%, bezogen auf das Gesamtgewicht der Zusammensetzung, vorliegt bzw. vorliegen.

16. Verfahren nach einem der vorhergehenden Ansprüche, **dadurch gekennzeichnet, dass** das Silikon bzw. die Silikone in einer Gesamtmenge im Bereich von 0,15 bis 10 Gew.-%, weiter bevorzugt von 0,3 bis 7 Gew.-%, weiter bevorzugt von 0,5 bis 5 Gew.-%, noch besser von 0,75 bis 4 Gew.-% und sogar noch besser von 1 bis 3 Gew.-%, bezogen auf das Gesamtgewicht der Zusammensetzung, vorliegt bzw. vorliegen.

17. Verfahren nach einem der vorhergehenden Ansprüche, **dadurch gekennzeichnet, dass** das Gewichtsverhältnis der Gesamtmenge von Copolymer bzw. Copolymeren von Crotonsäure oder Crotonsäure-Derivat zur Gesamtmenge von Silikon bzw. Silikonen im Bereich von 0,1 bis 15, weiter bevorzugt 0,5 bis 10 und noch besser von 1 bis 8 liegt.

18. Verfahren nach einem der vorhergehenden Ansprüche, **dadurch gekennzeichnet, dass** es außerdem einen Verdicker umfasst, der vorzugsweise aus vernetzten Copolymeren von Acryl- und/oder Methacrylsäure, vorzugsweise aus vernetzten Acrylsäure-Homopolymeren, ausgewählt ist.

19. Kosmetische Zusammensetzung, umfassend (i) ein Crotonsäure/Vinylacetat/Vinylneodecanoat-Terpolymer, (ii) ein oder mehrere Aminosilikone, wobei die Gesamtmenge an Aminosilikon im Bereich von 0,1 bis 15 Gew.-%, bezogen auf das Gesamtgewicht der Zusammensetzung liegt, und (iii) ein oder mehrere Pigmente; und wobei die Gesamtmenge an Silikon bzw. Silikonen im Bereich von 0,1 bis 15 Gew.-%, bezogen auf das Gesamtgewicht der Zusammensetzung, liegt.

20. Verwendung der Zusammensetzung nach Anspruch 19 zum Färben von Keratinfasern, insbesondere menschlichen Keratinfasern wie dem Haar.

## Revendications

1. Procédé pour la coloration de fibres kératiniques, en particulier de fibres kératiniques humaines telles que les cheveux, comprenant l'application auxdites fibres d'une composition comprenant (i) un ou plusieurs copolymères issus de la polymérisation d'au moins un monomère d'acide crotonique ou de dérivé d'acide crotonique et d'au moins un monomère d'ester de vinyle, (ii) une ou plusieurs aminosilicones, la quantité totale d'aminosilicone (s) se situant dans la plage de 0,1 % à 15 % en poids par rapport au poids total de la composition, et (iii) un ou plusieurs pigments ; et la quantité totale de silicone(s) se situant dans la plage de 0,1 % à 15 % en poids par rapport au poids total de la composition.

2. Procédé selon la revendication 1 **caractérisé en ce que** le ou lesdits copolymères sont issus de la polymérisation d'au moins un monomère d'acide crotonique et d'au moins un monomère d'ester de vinyle.

3. Procédé selon l'une quelconque des revendications précédentes, **caractérisé en ce que** ledit au moins un dérivé d'acide crotonique est choisi parmi des esters ou amides d'acide crotonique.

4. Procédé selon l'une quelconque des revendications précédentes, **caractérisé en ce que** ledit au moins un dérivé d'acide crotonique est choisi parmi les esters de l'acide crotonique de formule CH₃CH=CHCOOR'1 avec R'1 représentant une chaîne linéaire, ramifiée ou cyclique, saturée ou insaturée, éventuellement aromatique (aryle, aralkyle ou alkylaryle) à base de carbone et notamment à base d'hydrocarbure (alkyle), contenant 1 à 30 atomes de carbone, comprenant éventuellement une ou plusieurs fonctions choisies parmi -OH, -OR' avec R' alkyle en C1-C6 (alcoxy), -CN, -X (halogène, notamment Cl, F, Br ou I) tels que le crotonoate de méthyle et le crotonoate d'éthyle.

5. Procédé selon l'une quelconque des revendications précédentes **caractérisé en ce que** ledit au moins un dérivé d'acide crotonique est choisi parmi les amides de l'acide crotonique de formule CH₃CH=CHCONR'2R"2 avec R'2 et R"2, qui peuvent être identiques ou différents, représentant hydrogène ou une chaîne linéaire, ramifiée ou cyclique, saturée ou insaturée, éventuellement aromatique, à base de carbone et notamment à base d'hydrocarbure (alkyle), contenant 1 à 30 atomes de carbone, comprenant éventuellement une ou plusieurs fonctions choisies parmi -OH, -OR' avec R' alkyle en C1-C6 (alcoxy), -CN, -X (halogène, notamment Cl, F, Br ou I) .

6. Procédé selon les revendications 1 à 5, **caractérisé en ce que** le ou lesdits copolymères sont issus de la polymérisation d'au moins un monomère d'acide crotonique ou dérivé d'acide crotonique et d'au moins deux monomères d'ester de vinyle différents.

7. Procédé selon les revendications 1 à 6 **caractérisé en ce que** le ou lesdits monomères d'ester de vinyle sont choisis parmi l'acétate de vinyle, le propionate de vinyle, le butyrate (ou butanoate) de vinyle, l'éthylhexanoate de vinyle, le néononanoate de vinyle et le néododécanoate de vinyle, le néodécanoate de vinyle, le pivalate de vinyle, le cyclohexanoate de vinyle, le benzoate de vinyle, le 4-tert-butylbenzoate de vinyle et le trifluoroacétate de vinyle, préférablement parmi l'acétate de vinyle, le propionate de vinyle et le néodécanoate de vinyle, mieux encore parmi l'acétate de vinyle et le néodécanoate de vinyle.

8. Procédé selon les revendications 1 à 7 **caractérisé en ce que** le ou lesdits copolymères sont choisis parmi des copolymères issus de la polymérisation d'acide crotonique, d'acétate de vinyle et de propionate de vinyle, des copolymères issus de la polymérisation d'acide crotonique, d'acétate de vinyle et de néodécanoate de vinyle, et des mélanges correspondants.

9. Procédé selon les revendications 1 à 8 **caractérisé en ce que** le copolymère est un terpolymère d'acide crotonique/acétate de vinyle/néodécanoate de vinyle.

10. Procédé selon les revendications 1 à 9 **caractérisé en ce que** lesdits copolymères comprennent également d'autres monomères tels que des esters allyliques ou méthallyliques ou des éthers vinyliques.

11. Procédé selon l'une quelconque des revendications précédentes, dans lequel ledit copolymère d'acide crotonique issu de la polymérisation d'au moins un monomère d'acide crotonique ou de dérivé d'acide crotonique et d'au moins un monomère d'ester de vinyle est présent en une quantité dans la plage de 0,05 % à 15 % en poids par rapport au poids de la composition, préférablement de 0,1 % à 10 % en poids par rapport au poids de la composition, préférablement de 1 % à 5 % en poids par rapport au poids total de la composition.

12. Procédé selon l'une quelconque des revendications précédentes, **caractérisé en ce qu'**il contient au moins deux silicones différentes.

13. Procédé selon la revendication précédente, **caractérisé en ce qu'**il contient au moins une silicone dont la dénomination INCI est diméthicone.

14. Procédé selon l'une quelconque des revendications précédentes, caractérisé en que ladite amino silicone possède la dénomination INCI amodiméthicone.

15. Procédé selon l'une quelconque des revendications précédentes, **caractérisé en ce que** ladite ou lesdites silicone (s) sont présentes en une quantité totale d'au moins 0,15 %, plus préférentiellement d'au moins 0,3 %, plus préférablement d'au moins 0,5 %, mieux encore d'au moins 0,75 %, mieux encore de 1 % en poids par rapport au poids total de la composition.

16. Procédé selon l'une quelconque des revendications précédentes, **caractérisé en ce que** ladite ou lesdites silicone(s) sont présentes en une quantité totale dans la plage de 0,15 à 10 %, plus préférentiellement de 0,3 % à 7 % en poids, plus préférablement de 0,5 % à 5 % en poids, mieux encore de 0,75 % à 4 % en poids et même mieux encore de 1 % à 3 % en poids par rapport au poids total de la composition.

17. Procédé selon l'une quelconque des revendications précédentes, **caractérisé en ce que** le rapport pondéral de la quantité totale de copolymère (s) d'acide crotonique ou de dérivé d'acide crotonique sur la quantité totale de silicone(s) se situe dans la plage de 0,1 à 15, plus préférentiellement de 0,5 à 10 et mieux encore de 1 à 8.

18. Procédé selon l'une quelconque des revendications précédentes, **caractérisé en ce qu'**il comprend également un épaississant, préférablement choisi parmi des copolymères réticulés d'acide acrylique et/ou d'acide méthacrylique préférablement parmi des homopolymères d'acide acrylique réticulés.

19. Composition cosmétique comprenant (i) un terpolymère d'acide crotonique/acétate de vinyle/néodécanoate de vinyle, (ii) une ou plusieurs amino silicones, la quantité totale d'amino silicone se situant dans la plage de 0,1 % à 15 % en poids par rapport au poids total de la composition, et (iii) un ou plusieurs pigments ; et la quantité totale de silicone(s) se situant dans la plage de 0,1 % à 15 % en poids par rapport au poids total de la composition.

20. Utilisation de la composition selon la revendication 19, pour la coloration de fibres kératiniques, en particulier de fibres kératiniques humaines telles que les cheveux.
